# EUROPEAN PATENT APPLICATION

(11) **EP 1 258 261 A1**
(43) Date of publication of application: **20.11.2002**
(21) Application number: 01830309.9
(22) Date of filing: 15.05.2001
(51) Int. Cl.: A61M 1/30, A61M 5/32, A61M 5/158

(54) **Dialysis needle**

(71) Applicant: Catarsi Ing. Piero & C. S.r.l., 56012 Calcinaia (IT)
(72) Inventor: Santerini, Dante, 56021 Cascina (Pisa) (IT); Macchi, Romualdo, 56100 Pisa (IT); Catarsi, Piero, 56012 Calcinaia (Pisa) (IT); Pardini, Mario, 56019 Vecchiano (Pisa) (IT)
(74) Representative: Bardini, Marco Luigi

(57) **Abstract**

A dialysis needle (1A) having an end shaped as piercing tip generated by a cut (3) inclined with respect to the longitudinal axis of the needle and defining an opening (5) for the passage of blood. Near the piercing tip (1A) at least a side hole is provided (7) nearly opposite to the opening (5) and at least another hole (9; 10a,b) above the side hole (7) at a greater distance from the point of the piercing tip.

## Description

The present invention relates to a dialysis needle, intended in particular to eliminate some inconveniences that can occur during the aspiration phase of blood to be purified.

Dialysis needles are made substantially like needles for hypodermic injections or for intravenous injections. Namely, they are made up of a tubular barrel, an end of which is shaped as a piercing tip by means of a cut, inclined with respect to the needle axis, which defines an opening for the passage of blood. Compared to traditional needles, moreover, the dimensions of dialysis needles are much greater.

In particular, their total length measured from the point of the piercing tip to the engagement root is 25 mm with outside/inside diameter equal to 1.8/1.6 mm and the length of the piercing tip varies from 3 to 5 mm. The dialysis needle must be manufactured perfectly and during the production phase it always undergoes blast cleaning processes, siliconization and very accurate sterilization.

An inconvenience of the known dialysis needles consists in that the negative pressure, which is necessary to have a good pumping and therefore a remarkable quantity of blood available to the dialyzer in the unit time, creates a remarkable hydrodynamic unbalance, which causes the needle to move to an angled position with respect to the axis of the withdrawal vessel. In this way the opening of the piercing tip tends to draw alongside the wall of the vessel and to occlude said opening, because of the negative pressure, even making the vessel walls approach closer together. As a consequence the blood flux can be reduced till it stops.

The main object of the present invention is to provide a dialysis needle which allows to avoid said inconvenience and also to achieve other evident objects and advantages, which will be made clearer hereinafter.

This object is achieved with the dialysis needle according to the present invention, in which, near the conventionally pervious piercing tip, a first side hole is provided nearly opposite to the opening formed by the inclined cut, which generates the piercing tip, and at least a second hole formed upstream of said side hole, at a greater distance from the end of said piercing tip.

Said first hole is in front of the most distant part - with respect to the point of the piercing tip - of the opening created by the inclined cut of the piercing tip.

The two side holes can be aligned lengthwise or, preferably, the second hole can be made of a couple of diametrically opposed holes upstream of the first hole, angularly displaced, for example of 90°, and with a smaller diameter with respect to it.

The holes can have a diameter of the order of magnitude of, or slightly greater than, 2/3 of the inside diameter of the needle and anyway the diameter of the holes must be large enough, but has to be compatible with the strength that the residual cross section has to supply - for the intended use of the needle - by the or by each one of the side holes.

Further characteristics and advantages of the needle according to the present invention will be made clearer hereinafter with the following description of an embodiment thereof, made as an example but not limitative, referring to the attached drawing in which:
- figure 1 shows a partial longitudinal section view of the end shaped as a piercing tip of a dialysis needle according to the invention;
- figure 2 shows a view and a partial section of the needle of figure 1;
- figure 3 shows a cross section along lines III-III of figure 1;
- figure 4 shows a particular condition of the arrangement, inside a blood vessel, of the dialysis needle according to the invention in conditions of at least partial obstruction of the opening formed by the inclined cut of the tubular component of the needle, which generates the piercing tip;
- figure 5 shows a second embodiment of the needle according to the invention.

Referring to above mentioned figures, the needle at issue, indicated with 1, has a cylindrical wall with a circular section which is relatively thin as far as the strength required by the needle in use allows, considering in particular the side holes according to the invention, described hereinafter. A flat or, in case, curved, inclined cut, indicated with 3, forms the point 1A of the end of needle, shaped as a piercing tip, by means of which the wall of a vessel V, from which the blood to be purified is aspirated, has to be pierced (see figure 4).

The lumen, i.e. the cross section of vessel V, will be relatively larger than the cross section of needle 1 and usually, the aspiration takes place through the lengthened opening 5, which is delimited by the tubular wall of the needle where the cut 3 is made.

Because of the negative pressure exerted inside pervious needle 1, which is tubular, and considering the possible mutual positions of the vessel and of the piercing tip end of needle 1, lumen 5 can be frequently at least partially obstructed by the internal surface of the vessel V in a position shown in figure 4. In this condition, in the vicinity of the piercing tip 1A the vessel tends to approach the edge of opening 5 along the line of the inclined cut 3, generating the piercing tip, thus determining a reduction of the blood flow to be aspirated according to arrow f1 inside pervious needle 1. This obstacle to the free aspiration of blood flow towards the dialysis equipment can decrease the efficiency of the dialysis operation or at least increase the time required, this being discomfortable for the patient.

To avoid these inconveniences, according to the invention, a fist side hole 7 is provided in needle 1A, opposite to opening 5 and near point 1a of the piercing tip; the diameter of this hole 7 is relatively large, but smaller than the inside diameter of the needle, to obtain a quite large entry section, but a residual cross section of a sufficient mechanical strength.

Upstream of first hole 7, at a greater distance from point 1A, a couple of diametrically opposed holes 10a, 10b is provided, arranged sideways, but angularly displaced with respect to hole 7, for example of about 90°. The diameter of holes 10a, 10b is smaller than that of hole 7. As a mere example, the diameter of hole 7 can be 1.16 mm, whereas the diameter of holes 10a,b 0.6 mm. With this arrangement of the holes it is possible to obtain an optimum balance of the needle inside the vessel with respect to which it will maintain a central and parallel position, which will render the withdrawal more fluid and homogeneous, because it is not carried out by aspirating from the vessel area with the smallest blood flow, as occurs near the vessel walls.

With this disposition - in case opening 5, generated from inclined cut 3 of the needle end, which forms the point 1A, tends to remain obstructed from the internal surface of vessel V, as shown in figure 4 - a free section is certainly available for the inflow of blood aspirated through holes 7 and 10a,b according to arrow f0 of figure 4. Because of the given position of said holes 7 and 10a,b and because vessel V is practically not flattened, but tending to maintain its circular section elastically, in case of obstruction of opening 5, by approach to the wall of the vessel, there is a complete availability of flow through the holes 7 and 10a, b, which are distant from the wall of the vessel. On the contrary, in case holes 7 and/or 10a,b are obstructed because of an approach to the vessel wall, there is a complete availability of blood flow through opening 5 mentioned above. Therefore - with the shown arrangement - any possible obstacle to the circulation of blood according to arrow f1, due to the negative pressure inside needle 1 to feed dialysis equipment, is excluded.

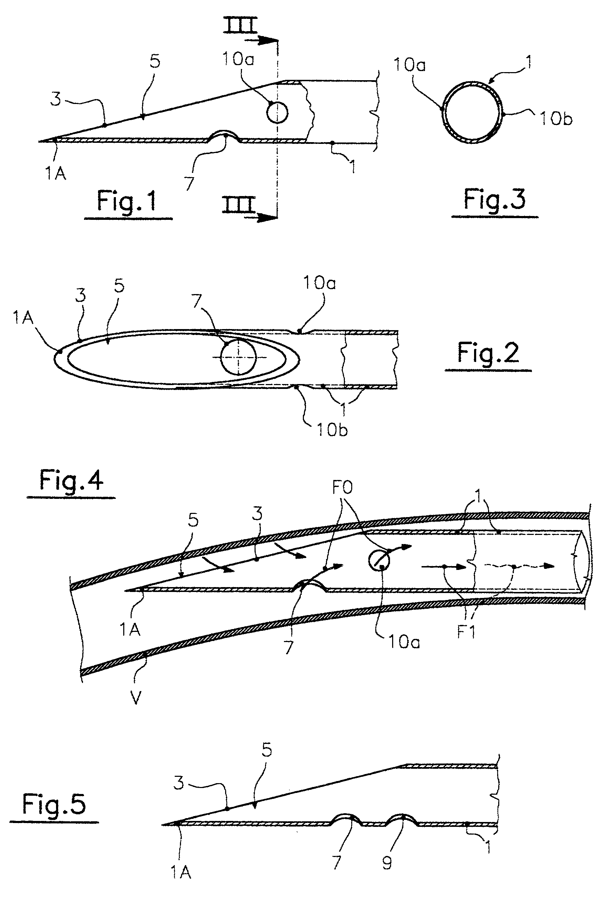

In the embodiment shown in figure 5, instead, a second side hole 9 is provided in front of the opening area 5, aligned lengthwise to hole 7 and more distant than hole 7 from the point 1a of the piercing tip.

With a view to ensuring a sufficient mechanical strength for the needle, a reduction of the diameter of hole 7 can be envisaged, if and when it is positioned in the area of the cut 3, which itself reduces the resistant section of the tubular material.

Of course, even if in the present description the withdrawal operation has been always referred to, the use of the needle according to the invention will be as advantageous also to reenter dialyzed blood in the bloodstream.

Variations and/or modifications can be brought to the dialysis needle according to the present invention, without departing from the scope of the invention itself, as defined in the attached claims.

## Claims

1. A dialysis needle, suited in particular for blood aspiration, comprising an pervious piercing tip (1A) generated by a cut (3) inclined with respect to the needle longitudinal axis, which defines an opening (5) for the passage of aspirated blood, **characterized in that** near the piercing tip (1A), a first side hole (7) is formed nearly opposite to the opening (5) generated by the inclined cut (3) which forms the piercing tip, and at least a second side hole (9; 10a, b) arranged upstream of said first hole and at a greater distance from the point of said piercing tip.

2. The dialysis needle according to claim 1, **characterized in that** said hole (7) is formed in front of the opening area (5) created by the inclined cut (3) of the piercing tip, which is more distant from the point(1A) of the piercing tip.

3. The dialysis needle according to claim 1 or 2, **characterized in that** said second hole comprises a couple of holes (10a,b) diametrically opposed and angularly displaced with respect to said first side hole(7).

4. The dialysis needle according to claim 3, in which the diameter of said couple of holes (10a, b) is smaller than the diameter of said side hole (7).

5. The dialysis needle according to any of the previous claims, in which said couple of holes (10a, b) is angularly displaced of 90° with respect to said side hole (7).

6. The dialysis needle according to any of the previous claims, in which the diameter of said holes, forming said couple of holes (10a, b), is smaller than that of said first hole (7).

7. The dialysis needle according to claim 1, **characterized in that** said second hole (9) is partially in front of the opening area (5) generated by the inclined cut (3) of the piercing tip, which is more distant from the point of the piercing tip (1A) .

8. The dialysis needle according to claim 7, **characterized in that** the two side holes (7, 9) are aligned lengthwise.

9. Dialysis needle according to at least one of the previous claims, **characterized in that** the diameter of the side holes (7, 9) is slightly greater than two thirties of the inside diameter of the needle.
